# EUROPÄISCHE PATENTANMELDUNG

(11) **EP 4 748 404 A1**
(43) Veröffentlichungstag der Anmeldung: **27.05.2026**
(21) Anmeldenummer: 24214808.8
(22) Anmeldetag: 22.11.2024
(51) Int. Cl.: A61L 2/22

(54) **VERFAHREN ZU EINER STEUERUNG EINES DEKONTAMINATIONSPROZESSES IN EINER KONTROLLIERTEN UMGEBUNG UND VERFAHREN ZUR BEWERTUNG EINES DEKONTAMINATIONSPROZESSES IN EINER KONTROLLIERTEN UMGEBUNG UND KORRESPONDIERENDE KONTROLLIERTE UMGEBUNG**

(71) Anmelder: SKAN AG, 4123 Allschwil (CH)
(72) Erfinder: Lehmann, Frank, 4102 Binningen (CH); Sommerhalder, Matthias, 4112 Bättwil (CH); Kemmerling, Dr. Simon, 4106 Therwil (CH); Krebsbach, Dr. Timo, 4310 Rheinfelden (CH); Novák, Martin, 4102 Binningen (CH); Eggert, Benjamin, 02763 Zittau (DE); Hommes, Dr. Gregor, 47669 Wachtendonk (DE)
(74) Vertreter: Mertzlufft-Paufler, Cornelius

(57) **Zusammenfassung**

Bei einem Dekontaminationsprozess wird somit erfindungsgemäß vorgeschlagen, wenigstens eine Umweltgröße automatisiert zu erfassen, um eine Zuführung eines Dekontaminationsmittels (7) in die kontrollierte Umgebung (1) automatisiert zu regeln.

## Beschreibung

Die Erfindung betrifft ein Verfahren zu einer Steuerung eines Dekontaminationsprozesses in einer kontrollierten Umgebung.

Die Erfindung betrifft weiter ein Verfahren zur Bewertung eines Dekontaminationsprozesses in einer kontrollierten Umgebung, wobei während des Dekontaminationsprozesses eine Zuführung eines Dekontaminationsmittels in die kontrollierte Umgebung gesteuert wird.

Kontrollierte Umgebungen sind bekannt und können beispielsweise dadurch charakterisierbar sein, dass Zustandsparameter, beispielsweise Druck, Temperatur, Luftzusammensetzung, Luftströmungsgeschwindigkeit und/oder Luftfeuchte, und/oder ein Stoffaustausch der Umgebung mit deren Außenwelt kontrolliert definierbar ist/sind. Eine Liste von Beispielen für kontrollierte Umgebungen umfassen Containments, insbesondere Isolatoren und Gloveboxes, und Restricted Access Barrier Systems (Zugangsbeschränktes Barrieresystem, RABS), insbesondere vom offenen oder geschlossenen Typ. Kontrollierte Umgebungen werden beispielsweise eingesetzt, um während eines vorzugsweise industriellen Prozesses eine unerwünschte Wechselwirkung mit der Außenwelt zu vermindern oder zu eliminieren. Ein beispielhafter Anwendungsfall kann das Abfüllen oder Umpacken eines Medikaments sein, ein anderer eine sterile Montage eines Applikators für eine Medikament.

Zur Vorbereitung der kontrollierten Umgebung wird häufig ein Dekontaminationsprozess durchgeführt, durch welchen vermehrungsfähige Verunreinigungen biologisch deaktiviert werden. Hierzu wirkt ein Dekontaminationsmittel auf die kontrollierte Umgebung ein. Als Dekontaminationsmittel sind beispielsweise lebensfeindliche Substanzen wie Wasserstoffperoxid (oder Wasserstoffperoxid-haltige Gemische) oder lebensfeindliche und/oder ionisierende Strahlungen wie UV-und/oder Gamma-Strahlung und/oder hochenergetische Teilchen-Strahlung verwendbar.

Der Erfindung liegt die Aufgabe zugrunde, den Dekontaminationsprozess in kontrollierten Umgebungen zu verbessern.

Zur Lösung der genannten Aufgabe sind erfindungsgemäß die Merkmale von Anspruch 1 vorgesehen. Insbesondere wird somit zur Lösung der genannten Aufgabe erfindungsgemäß vorgeschlagen, dass wenigstens eine Umweltgröße in der kontrollierten Umgebung erfasst und zu einer automatischen Ansteuerung einer Zuführung des Dekontaminationsmittels in die kontrollierte Umgebung verwendet wird. Von Vorteil ist dabei, dass ein Dekontaminationsprozess optimierbar ist. Unnötig lange Dekontaminationsprozesse sind vermeidbar. Ein Dekontaminationsprozess ist somit einfach und automatisch an geänderte Umgebungsbedingungen (Beladung, Wetterlage, Aufstellungsort) anpassbar.

Allgemein kann die Umweltgröße beispielsweise zumindest eine mit einer Konzentration des Dekontaminationsmittels in der kontrollierten Umgebung korrelierende Konzentrations-Messgröße sein. Beispielsweise hierfür umfassen eine Konzentration des Dekontaminationsmittels, einen Partialdruck des Dekontaminationsmittels und/oder eine Sättigung des Dekontaminationsmittels und/oder eine Kondensationstemperatur (Taupunkt) des Dekontaminationsmittels.

Hierbei kann die Zuführung mit positivem oder negativem Masseeintrag, z.B. durch ein Versprühen oder Verdampfen des Dekontaminationsmittels oder durch ein Spülen der kontrollierten Umgebung mit einem Dekontaminationsmittel-armen oder -freien Fluid, angesteuert werden.

Beispiele für eine Konzentrations-Messgröße umfassen Sättigung und Konzentration des Dekontaminationsmittel in einem aufnehmenden Gas.

Insbesondere kann vorgesehen sein, dass die Umweltgröße mit wenigstens einem frei aufgestellten Messgerät erfasst wird. Hierbei kann eine freie Aufstellung beispielsweise dadurch charakterisierbar sein, dass das Messgerät (oder zumindest dessen Sensor) im Normalbetrieb von einer in der kontrollierten Umgebung angeworfenen Laminarströmung umspült wird und/oder dass das Messgerät (oder zumindest dessen Sensor) das Dekontaminationsmittel während des Dekontaminationsprozesse hauptsächlich durch Diffusion erhält. Von Vorteil ist dabei, dass die zu dekontaminierenden Oberflächen aus nächster Nähe überwacht und für eine Steuerung des Dekontaminationsprozesses verwendet werden können.

Besonders günstig ist es, wenn die Umweltgröße (beispielsweise als ggf. gewichteter Mittelwert) aus Signalen von mehr als einem Messgerät gebildet werden kann. Dies ermöglicht eine genauere örtliche Abbildung der Verhältnisse in der kontrollierten Umgebung. Die Umweltgröße kann auch mehrere Komponenten aufweisen, um eine Ortsauflösung abzubilden.

Die Zuführung des Dekontaminationsmittels kann beispielsweise durch Versprühen und/oder Vernebeln erfolgen. Ein Aufheizen des Dekontaminationsmittels - wie beispielsweise für ein Verdampfen - ist verzichtbar. Von Vorteil ist dabei, dass sich der Prozess näher an den Sättigungsbereich nähern kann, da hier keine heiße Gasphase erzeugt wird. Dies ermöglicht eine besonders effiziente Dekontamination. Bevorzugt ist hierzu eine Düse, beispielsweise eine Einstoffdüse oder eine Zweistoffdüse, vorgesehen.

Es kann somit vorgesehen sein, dass ein Aerosol das das Dekontaminationsmittel enthält, erzeugt und verwendet wird. Dies kann in einer Weiterbildung dazu genutzt werden, dass die die Konzentrations-Messgröße in einem Sättigungsbereich oder alternativ in einem Bereich, in dem das Konzentrationsrisiko an den Wänden der kontrollierten Umgebung unterhalb eines vordefinierten Wertes liegt, gehalten wird.

Bei einer Ausgestaltung der Erfindung kann vorgesehen sein, dass eine Zufuhrrate des Dekontaminationsmittels automatisiert erhöht wird, wenn die Konzentrations-Messgröße unter einem Sollwert liegt. Somit ist ein Nachführen bei einem höheren Bedarf an Dekontaminationsmittel einfach automatisch erreichbar.

Bei einer Ausgestaltung der Erfindung kann vorgesehen sein, dass die Umweltgröße wiederkehrend erfasst wird. Somit sind Zeitverläufe erfassbar und Prozesse überwachbar.

Bei einer Ausgestaltung der Erfindung kann vorgesehen sein, dass die erfasste Umweltgröße an die Ansteuerung rückgekoppelt wird. Somit ist eine direkte Einwirkung auf eine Zuführung von Dekontaminationsmittel erreichbar.

Bei einer Ausgestaltung der Erfindung kann vorgesehen sein, dass die Umweltgröße zur automatisierten Auswahl eines Ansteuerungsschemas verwendet wird. Somit sind weitere Bedingungen wie Luftfeuchte, Lufttemperatur, Luftdruck oder sonstige, relevante Faktoren für den Dekontaminationsprozess berücksichtigbar.

Bei einer Ausgestaltung der Erfindung kann vorgesehen sein, dass eine Zuführung des Dekontaminationsmittels derart angesteuert wird, dass eine Sättigung von mehr als 80% oder mehr als 90% oder mehr als 95% oder eine Übersättigung erreicht wird. Dies ermöglich eine möglichst effiziente Dekontamination.

Bei einer Ausgestaltung der Erfindung kann vorgesehen sein, dass ein Sensor, insbesondere der bereits erwähnte Sensor, der die Umweltgröße erfasst, mit einem aufgeheizten Messgas versorgt wird, um diese Flüssigkeitsphasen des Aerosols aufzuschließen. Wird ein Messignal dieses Sensors mit einem unaufgeheizten Sensor verglichen, so kann berechnet werden, welcher Anteil von H2O2 in der Flüssigkeitsphase des Aerosols liegt und welche in der Gasphase. Diese Differenz kann zur Regelung des Kreislaufes genutzt werden, was ebenfalls beansprucht werden soll. Dies hat den Vorteil, dass ein Regelungspunkt sehr nahe an den Sättigungspunkt gelegt werden kann, und die Sensoranordnung auch bei einem di Sättigungsgrenze überschießenden Regelungskreislauf nicht blind wird.

Bei einer Ausgestaltung der Erfindung kann vorgesehen sein, dass eine Zufuhrrate des Dekontaminationsmittels automatisiert vermindert wird, wenn die Umweltgröße über einem Sollwert liegt. Somit ist eine unnötig hohe Zuführung von Wasserstoffperoxid vermeidbar. Dies kann eine nachfolgende Spülung vereinfachen und/oder verkürzen.

Bei einer Ausgestaltung der Erfindung kann vorgesehen sein, dass die Umweltgröße mit einem zeitabhängigen Sollwert automatisiert verglichen wird. Somit sind beliebige Steuerkurven, insbesondere Rampen, realisierbar.

Bei einer Ausgestaltung der Erfindung kann vorgesehen sein, dass der Dekontaminationsprozess beendet wird, wenn ein vorbestimmtes Kriterium für eine mit der Umweltgröße korrelierende Wirksamkeitsaussage erfüllt ist. Somit ist ein unnötiges Weiterlaufen des Dekontaminationsprozesses vermeidbar.

Alternativ oder zusätzlich werden erfindungsgemäß zur Lösung der genannten Aufgabe die Merkmale das zweiten nebengeordneten Verfahrensanspruch vorgeschlagen. Insbesondere wird somit zur Lösung der genannten Aufgabe bei einem Verfahren zur Bewertung eines Dekontaminationsprozesses in einer kontrollierten Umgebung der eingangs beschriebenen Art erfindungsgemäß vorgeschlagen, dass eine mit einer Konzentration des Dekontaminationsmittels in der kontrollierten Umgebung korrelierende Konzentrations-Messgröße erfasst wird und eine Bewertungsinformation aus einer Verarbeitung der Konzentrations-Messgröße und einer durch die Steuerung der Zuführung vorgegebene Sollgröße automatisiert ermittelt wird. Somit ist eine ordnungsgemäße Durchführung der Dekontamination automatisch protokollierbar. Abweichungen sind einfach und manipulationssicher erfassbar.

Bei einer Ausgestaltung der Erfindung kann vorgesehen sein, dass die Sollgröße einen unter vorbestimmten Umweltbedingungen herrschenden Wert der Konzentrations-Messgröße beschreibt. Somit ist ein direkter Vergleich dieser relevanten Messgröße erreichbar.

Bei einer Ausgestaltung der Erfindung kann vorgesehen sein, dass die Sollgröße aus einer Massenbilanz an Dekontaminationsmittel automatisiert bestimmt wird. Somit ist ein Einwirkung des Dekontaminationsmittels einfach vorgebbar und/oder erreichbar.

Bei einer Ausgestaltung der Erfindung kann vorgesehen sein, dass ein zeitlicher Verlauf der Konzentrations-Messgröße mit einem zeitlichen Verlauf der Sollgröße verglichen wird. Somit ist eine kontinuierliche Kontrolle einer abweichungsfreien Durchführung möglich.

Bei einer Ausgestaltung der Erfindung kann vorgesehen sein, dass eine Fehlermeldung generiert wird, wenn die Konzentrations-Messgröße außerhalb eines durch die Sollgröße vorgegebenen Wertebereichs liegt. Somit sind Abweichungen automatisch protokollierbar.

Bei einer Ausgestaltung der Erfindung kann vorgesehen sein, dass eine Fehlermeldung generiert wird, wenn eine zeitliche Änderung der Konzentrations-Messgröße außerhalb eines für eine zeitliche Änderung der Sollgröße vorgegebenen Wertebereichs liegt. Somit sind Toleranzbereiche einrichtbar.

Eine bevorzugte Anwendung der Erfindung von möglicherweise eigenständiger erfinderischer Qualität sieht bei einem Verfahren zur Funktionsprüfung einer Dekontaminationsvorrichtung einer kontrollierten Umgebung vor, dass ein erfindungsgemäßes Verfahren, insbesondere wie zuvor beschrieben und/oder nachstehend beansprucht, durchgeführt wird und dass eine Fehlermeldung ausgegeben wird, wenn die Bewertungsinformation ein vorbestimmtes Kriterium nicht erfüllt. Somit ist eine ordnungsgemäße Durchführung der Dekontamination automatisch erfassbar und/oder dokumentierbar.

Erfindungsgemäß ist weiter eine kontrollierte Umgebung mit Mitteln zur Ausführung eines erfindungsgemäßen Verfahrens, insbesondere wie zuvor beschrieben und/oder nachstehend beansprucht, vorgesehen.

Beispielsweise kann die kontrollierte Umgebung als Containment oder als Isolator und/oder mit einer Steuervorrichtung, die zur Ausführung eines erfindungsgemäßen Verfahrens, insbesondere wie zuvor beschrieben und/oder wie nachstehend beansprucht, eingerichtet ist.

Die Erfindung wird nun anhand eines Ausführungsbeispiels näher beschrieben, ist aber nicht auf dieses Ausführungsbeispiel beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigt
- Figur 1: eine schematische Darstellung einer kontrollierten Umgebung zur Ausführung der erfindungsgemäßen Verfahren,
- Fig. 2: eine schematische Darstellung eines Regelkreises der kontrollierten Umgebung nach Fig. 1 und
- Fig. 3: Kurven von Soll- und Messwerten der Umweltgröße zur Erläuterung der Erfindung, wobei auf der y-Achse die Zeit und auf der y-Achse die Messwerte aufgetragen sind.

Eine im Ganzen mit Ziffer 1 bezeichnete kontrollierte Umgebung - hier beispielhaft als Isolator dargestellt - weist eine äu-βere Begrenzung 2 auf, die einen Innenraum 3 gegenüber der Au-βenwelt 4 derart abgrenzt, dass ein Stoffaustausch zwischen dem Innenraum 3 und der Außenwelt 4 nur kontrolliert stattfindet. Die äußere Begrenzung 2 kann ganz oder teilweise aus Glas oder Metall gefertigt sein und Schleusen, Türen, Ports, Handschuheingriffe und weitere Mittel zur Manipulation und/oder zum kontrollierten Waren- und/oder Stoffaustausch aufweisen, die hier zur Vereinfachung weggelassen sind.

Die kontrollierte Umgebung 1 ist mit einer Luftzufuhr 5 in an sich bekannter Weise ausgerüstet, die typischerweise in einem Deckenbereich der äußeren Begrenzung 2 angeordnet ist.

Durch die Luftzufuhr 5 gelangt gereinigte Luft 6 in den Innenraum 3. Typischerweise erfolgt dies derart, dass eine laminare Luftströmung und/oder eine Luftströmung mit einer Vorzugsrichtung von oben nach unten gebildet ist.

Zur Vorbereitung der kontrollierten Umgebung 1 wird ein Dekontaminationsprozess durchgeführt, bei dem ein Dekontaminationsmittel 7 mit einer Dekontaminationsvorrichtung 8 in den Innenraum 3 eingebracht wird.

Dies kann beispielsweise durch Zerstäuben des Dekontaminationsmittels 7 oder durch Verdampfen des Dekontaminationsmittels 7 erfolgen, so dass im Ergebnis das Dekontaminationsmittel 7 als Aerosol oder Gas im Innenraum 3 vorliegt, wo es auf Objekte 9 zur Dekontamination einwirken kann.

Der Dekontaminationsprozess kann beispielsweise zu einer Deaktivierung von biologisch vermehrungsfähigem Material, insbesondere Mikroorganismen, führen.

Die Objekte 9 können hierbei allgemein zur Ausführung eines beispielsweise pharmazeutischen Prozesses in der kontrollierten Umgebung 1 im Anschluss an den Dekontaminationsprozess durchgeführt werden soll.

Eine nicht abschließende Liste von möglichen Objekten 9 sind Sortiertöpfe, Behälter, Transportmittel, Instrumente und/oder Verbrauchsmaterialien.

Die kontrollierte Umgebung 1 weist weiter eine Sensoreinheit 10 auf, mit der ein Teil 11 der dekontaminationsmittelhaltigen Raumluft mit einem Sensor 12 in Kontakt bringbar ist. Die Sensoreinheit 10 ist hierbei als ein Messgerät 24 frei aufgestellt, so dass die Raumluft frei - beispielsweise über Diffusion - an den Sensor 12 gelangen kann.

Mit dem Sensor 12 ist wenigstens eine Umweltgröße erfassbarer.

Im Ausführungsbeispiel ist diese Umweltgröße wenigstens eine mit einer Konzentration des Dekontaminationsmittels 7 in der kontrollierten Umgebung 1 korrelierende Konzentrations-Messgröße. Beispiele hierfür sind eine Konzentration des Dekontaminationsmittels 7 in der Raumluft oder ein Partialdruck des Dekontaminationsmittels 7 oder eine Sättigung des Dekontaminationsmittels 7 in der Raumluft. Hierzu kann die Sensoreinheit 10 weitere Sensoren, insbesondere für Temperatur, Luftfeuchtigkeit, Luftdruck und andere Messgrößen, aufweisen.

Die kontrollierte Umgebung 1 weist außerdem eine Steuervorrichtung 13 auf. Die Steuervorrichtung 13 steuert die Dekontaminationsvorrichtung 8 über eine Steuerleitung 14 an, um die Zuführung des Dekontaminationsmittels 7, insbesondere eine Zufuhrrate des Dekontaminationsmittels 7, automatisiert zu definieren.

Über eine Steuerleitung 15 kann die Steuervorrichtung 13 die Luftzufuhr 5 ansteuern, insbesondere zu einer Druckregelung und/oder zu einer Regelung einer Strömungsgeschwindigkeit im Innenraum 3.

Über eine Steuerleitung 16 erhält die Steuervorrichtung 13 Messwerte der erwähnten Umweltgröße und gegebenenfalls weitere Umweltgrößen von der Sensoreinheit 10.

Es ist auch denkbar, weitere Sensoreinheiten 10 im Innenraum 3, insbesondere an kritischen Orten, aufzustellen und mit der Steuervorrichtung 13 zu verbinden.

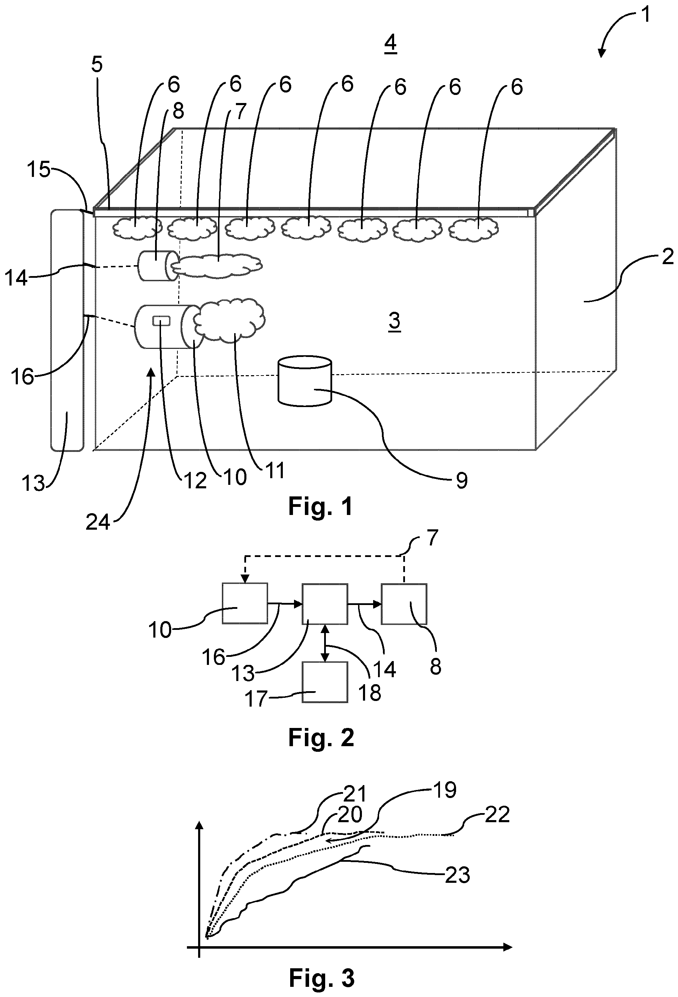

Somit ist ein Regelkreis gebildet, dessen Grundzüge in Figur 2 dargestellt sind.

Die Steuervorrichtung 13 ist mit einem Speicher 17 über eine Datenverbindung 18 verbunden.

In dem Speicher 17 sind Sollwerte für die Umweltgröße, insbesondere für die Konzentrations-Messgröße, hinterlegt. Diese Sollwerte können zeitabhängig oder zeitunabhängig für den Dekontaminationsprozess vorgegeben sein. Beispielsweise kann so eine Rampe für die Konzentrations-Messgröße definiert sein. Fig. 3 zeigt beispielhaft mehrere Kurven 20, 21, 22 für Sollwerte in einem Zeitdiagramm (x-Achse).

Die Steuervorrichtung 13 empfängt über die Steuerleitung 16 Messwerte der Umweltgröße von der Sensoreinheit 10. Hierzu wird die Umweltgröße wiederkehrend erfasst.

Die Steuervorrichtung 13 vergleicht diese Messwerte mit der bereits erwähnten Sollgröße und gibt in Abhängigkeit von dem Vergleich über die Steuerleitung 14 eine Ansteuerung an die Dekontaminationsvorrichtung 8, um die Zuführung des Dekontaminationsmittels 7 so einzustellen, dass die künftigen Messwerte näher an dem Sollwert liegen.

Dies geschieht dadurch, dass das Dekontaminationsmittel 7 auf die Sensoreinheit 10 einwirkt. Beispielsweise kann eine Zerstäubungsrate und/oder eine Verdampfungsrate automatisch angepasst werden.

Beispielsweise kann eine Zufuhrrate des Dekontaminationsmittels 7 automatisiert erhöht werden, wenn die Konzentrations-Messgröße unter einem Sollwert liegt, oder die Zuführrate des Dekontaminationsmittels 7 kann erniedrigt werden, wenn die Konzentrations-Messgröße über dem Sollwert liegt.

Hierbei weist die Steuervorrichtung 13 nicht weiter dargestellte Zeitmessmittel auf, um die Messwerte der Umweltgröße mit dem jeweils gültigen zeitabhängigen Sollwert zu vergleichen.

Aus den gemessenen Umweltgrößen, insbesondere aus der Konzentrations-Messgröße, einer Feuchte-Messgröße, die mit einer Luftfeuchtigkeit im Innenraum 3 korreliert, einer Temperatur-Messgröße, die mit einer Temperatur im Innenraum 3 korreliert, und der Zeit kann eine Wirksamkeitsaussage für den Dekontaminationsprozess zeitlich wiederkehrend ermittelt werden, insbesondere beispielsweise eine Anzahl von verbleibenden vermehrungsfähigen Materialien, insbesondere Mikroorganismen, durch den bisherigen Dekontaminationsprozess.

In dem Speicher 17 kann ein Sollwert für die Wirksamkeitsaussage hinterlegt werden, nach dessen Erreichen die Steuervorrichtung 13 den Dekontaminationsprozess beendet.

Mit der Steuervorrichtung ist somit ein Verfahren zur Steuerung eines Dekontaminationsprozesses in der beschriebenen Weise ausführbar.

Die Steuervorrichtung ist außerdem noch zu einem Verfahren zur Bewertung eines Dekontaminationsprozesses eingerichtet, wie im Folgenden näher beschrieben wird.

Hierzu zeigt Figur 3 allgemein schematisch einen zeitlichen Verlauf eines Sollwerts für die genannte Umweltgröße, insbesondere für die Konzentrations-Messgröße. Dieser zeitliche Verlauf kann beispielsweise in dem Speicher 17 in Form von Kurven 20, 21, 22 hinterlegt sein. Die genaue Zahl der Kurven 20, 21, 22 ist frei wählbar.

Der konkrete zeitliche Verlauf ist hierbei so gewählt, dass ein gewünschter Dekontaminationsprozess sicher zum gewünschten Deaktivieren oder Abtöten des biologisch vermehrungsfähigen Materials führt.

Wird der Dekontaminationsprozess nun durchgeführt, kann es passieren, dass die Messwerte der Konzentrations-Messgröße den durchgezogenen Verlauf der Figur 3 ergeben. Offensichtlich gibt es hiermit eine Abweichung von dem Sollverlauf nach einem der Kurven 20, 21, 22.

Die Steuerungsvorrichtung 13 ist hier nun so eingerichtet, dass sie aus der Abweichung eine Bewertungsinformation ableitet, welche den konkret durchgeführten Dekontaminationsprozess charakterisieren kann.

Ist die Abweichung so groß, dass die Konzentrations-Messgröße außerhalb eines für die Sollgröße vorgegebenen Wertebereichs 19 läuft (wie in Fig. 3), so liegt ein Fehler vor.

Beispielsweise kann die Konzentration des in der Dekontaminationsvorrichtung 8 vorgehaltenen Dekontaminationsmittels 7 fehlerhaft sein, oder es kann eine unzulässige Beladung des Innenraums 3 mit Objekten 9 vorliegen oder es kann ein vorgeschalteter Reinigungsvorgang für die kontrollierte Umgebung 1 unsachgemäß ausgeführt worden sein, oder die sonstigen Umweltbedingungen können so abweichend sein, dass der Dekontaminationsprozess angepasst werden muss. Es sind auch weitere Fehlerquellen denkbar, zum Beispiel die Verwendung von Substanzen, die das Dekontaminationsmittel unerwünscht binden oder umwandeln.

Hierbei können in dem Speicher 17 mehrere Kurven 20, 21, 22 für die Sollgröße hinterlegt sein, die in Abhängigkeit von Umweltgrößen wie beispielsweise Temperatur, Luftdruck und dergleichen, und/oder in Abhängigkeit von einer Beladung des Innenraums 3 automatisiert ausgewählt werden können. Für jede Kurve 20, 21, 23 ergibt sich daher ein anderes Ansteuerungsschema. Bei weiteren Ausführungsbeispielen werden Koeffizienten des Regelkreises (bei gleichbleibender oder veränderter Kurve 20, 21, 22) an geänderte Umweltgrößen angepasst.

Der bereits erwähnte Wertebereich 19 kann sich beispielsweise als Bereich zwischen zwei derartigen Kurven 20, 22 für die Sollgröße erstrecken.

Auf diese Weise kann ein Verfahren zur Funktionsprüfung einer Dekontaminationsvorrichtung 8 in einer kontrollierten Umgebung 1 ausgeführt werden, wobei eine Fehlermeldung ausgegeben wird, wenn die Bewertungsinformation ein vorbestimmtes Kriterium nicht erfüllt. Dies kann beispielsweise dann sein, wenn die gemessene Kurve 23 der Konzentrations-Messgröße außerhalb eines zulässigen Wertebereichs 19 liegt.

Das Messgerät 24 kann in einem Ausführungsbeispiel einen Sensor 12 enthalten, dem aufgeheiztes Messgas zugeführt wird. Hierzu kann eine an sich bekannte Heizvorrichtung vorgesehen sein. Wird das Aufheizen so eingestellt, dass ein Aufschluss der Flüssigkeitsphase des Dekontaminationsmittels im Messgas (Aerosol) erfolgt, so kann durch einen Vergleich der Messsignale dieses Sensors mit denen eines unaufgeheizten Sensors ein Anteil des Dekontaminationsmittels in der Flüssigkeitsphase ermittelt werden.

Bei einem Dekontaminationsprozess wird somit erfindungsgemäß vorgeschlagen, wenigstens eine Umweltgröße automatisiert zu erfassen, um eine Zuführung eines Dekontaminationsmittels 7 in die kontrollierte Umgebung 1 automatisiert zu regeln.

### Bezugszeichenliste

- 1: kontrollierte Umgebung
- 2: äußere Begrenzung
- 3: Innenraum
- 4: Außenwelt
- 5: Luftzufuhr
- 6: Luft
- 7: Dekontaminationsmittel
- 8: Dekontaminationsvorrichtung
- 9: Objekt
- 10: Sensoreinheit
- 11: Teil der Raumluft
- 12: Sensor
- 13: Steuervorrichtung
- 14: Steuerleitung
- 15: Steuerleitung
- 16: Steuerleitung
- 17: Speicher
- 18: Datenverbindung
- 19: Wertebereich
- 20: Kurve der Sollgröße
- 21: Kurve der Sollgröße
- 22: Kurve der Sollgröße
- 23: Kurve der Konzentrations-Messgröße
- 24: Messgerät

## Patentansprüche

1. Verfahren zu einer Steuerung eines Dekontaminationsprozesses in einer kontrollierten Umgebung (1), **dadurch gekennzeichnet, dass** wenigstens eine Umweltgröße, insbesondere eine mit einer Konzentration des Dekontaminationsmittels in der kontrollierten Umgebung (1) korrelierende Konzentrations-Messgröße, in der kontrollierten Umgebung (1) vorzugsweise mit wenigstens einem frei aufgestellten Messgerät (24) erfasst und zu einer automatischen Ansteuerung einer Zuführung, insbesondere durch Versprühen und/oder Vernebeln, des Dekontaminationsmittels (7) in die kontrollierte Umgebung (1) verwendet wird.

2. Verfahren nach Anspruch 1, **dadurch gekennzeichnet, dass** eine Zufuhrrate des Dekontaminationsmittels (7) automatisiert erhöht wird, wenn die Umweltgröße, insbesondere die Konzentrations-Messgröße, unter einem Sollwert liegt.

3. Verfahren nach einem der vorangehenden Ansprüche, **dadurch gekennzeichnet, dass** die Umweltgröße wiederkehrend erfasst wird und/oder dass die erfasste Umweltgröße an die Ansteuerung rückgekoppelt wird.

4. Verfahren nach einem der vorangehenden Ansprüche, **dadurch gekennzeichnet, dass** die Umweltgröße zur automatisierten Auswahl eines Ansteuerungsschemas verwendet wird und/oder dass eine Zuführung des Dekontaminationsmittels (7) derart angesteuert wird, dass eine Sättigung von mehr als 80% oder mehr als 90% oder mehr als 95% oder eine Übersättigung erreicht wird.

5. Verfahren nach einem der vorangehenden Ansprüche, **dadurch gekennzeichnet, dass** der oder ein die Umweltgröße erfassender Sensor (12) mit einem aufgeheizten Messgas versorgt wird, insbesondere wobei ein Messignal dieses Sensors (12) mit dem eines unaufgeheizten Sensors verglichen wird.

6. Verfahren nach einem der vorangehenden Ansprüche, **dadurch gekennzeichnet, dass** eine Zufuhrrate des Dekontaminationsmittels (7) automatisiert vermindert wird, wenn die Umweltgröße über einem Sollwert liegt.

7. Verfahren nach einem der vorangehenden Ansprüche, **dadurch gekennzeichnet, dass** die Umweltgröße mit einem zeitabhängigen Sollwert automatisiert verglichen wird.

8. Verfahren nach einem der vorangehenden Ansprüche, **dadurch gekennzeichnet, dass** der Dekontaminationsprozess beendet wird, wenn ein vorbestimmtes Kriterium für eine mit der Umweltgröße korrelierende Wirksamkeitsaussage erfüllt ist.

9. Verfahren zur Bewertung eines Dekontaminationsprozesses in einer kontrollierten Umgebung (1), wobei während des Dekontaminationsprozesses eine Zuführung eines Dekontaminationsmittels in die kontrollierte Umgebung (1) gesteuert wird, **dadurch gekennzeichnet, dass** eine mit einer Konzentration des Dekontaminationsmittels (7) in der kontrollierten Umgebung (1) korrelierende Konzentrations-Messgröße erfasst wird und eine Bewertungsinformation aus einer Verarbeitung der Konzentrations-Messgröße und einer durch die Steuerung der Zuführung vorgegebene Sollgröße automatisiert ermittelt wird.

10. Verfahren nach einem der vorangehenden Ansprüche, **dadurch gekennzeichnet, dass** die Sollgröße einen unter vorbestimmten Umweltbedingungen herrschenden Wert der Konzentrations-Messgröße beschreibt.

11. Verfahren nach einem der vorangehenden Ansprüche, **dadurch gekennzeichnet, dass** die Sollgröße aus einer Massenbilanz an Dekontaminationsmittel (7) automatisiert bestimmt wird.

12. Verfahren nach einem der vorangehenden Ansprüche, **dadurch gekennzeichnet, dass** ein zeitlicher Verlauf der Konzentrations-Messgröße mit einem zeitlichen Verlauf der Sollgröße verglichen wird.

13. Verfahren nach einem der vorangehenden Ansprüche, **dadurch gekennzeichnet, dass** eine Fehlermeldung generiert wird, wenn die Konzentrations-Messgröße außerhalb eines durch die Sollgröße vorgegebenen Wertebereichs (19) liegt.

14. Verfahren nach einem der vorangehenden Ansprüche, **dadurch gekennzeichnet, dass** eine Fehlermeldung generiert wird, wenn eine zeitliche Änderung der Konzentrations-Messgröße außerhalb eines für eine zeitliche Änderung der Sollgröße vorgegebenen Wertebereichs liegt.

15. Verfahren zur Funktionsprüfung einer Dekontaminationsvorrichtung einer kontrollierten Umgebung (1), **dadurch gekennzeichnet, dass** ein Verfahren nach einem der vorangehenden Ansprüche durchgeführt wird und dass eine Fehlermeldung ausgegeben wird, wenn die Bewertungsinformation ein vorbestimmtes Kriterium nicht erfüllt.

16. Kontrollierte Umgebung (1), insbesondere Isolator, mit Mitteln zur Ausführung eines Verfahrens nach einem der vorangehenden Ansprüche.
